# EUROPÄISCHE PATENTSCHRIFT

(11) **EP 1 026 148 B1**
(45) Veröffentlichungstag und Bekanntmachung des Hinweises auf die Patenterteilung: **07.05.2003**
(21) Anmeldenummer: 00100310.2
(22) Anmeldetag: 07.01.2000
(51) Int. Cl.: C07C 69/38, C07C 67/11

(54) **Verfahren zur Herstellung von Malonsäureestern**
Process for the preparation of esters of malonic acid
Procédé de préparation d'esters d'acide malonique

(30) Priorität: 11.01.1999 EP 99100411
(43) Veröffentlichungstag der Anmeldung: 09.08.2000
(73) Patentinhaber: Lonza AG, 4052 Basel (CH)
(72) Erfinder: Hanselmann, Paul, 3902 Brig-Glis (CH); Hildbrand, Stefan, 3930 Visp (CH)

(56) Entgegenhaltungen:
- EP-A- 0 534 817
- JAIME DE LA ZERDA ET AL.: "Selective Monoetherification and Monoesterification of Diols and Diacids under Phase-Transfer Conditions" TETRAHEDRON., Bd. 45, Nr. 5, 1989, Seiten 1533-1536, XP002138240 ELSEVIER SCIENCE PUBLISHERS, AMSTERDAM., NL ISSN: 0040-4020

## Beschreibung

Die Erfindung betrifft ein Verfahren zur Herstellung von Malonsäurediestern der allgemeinen Formel 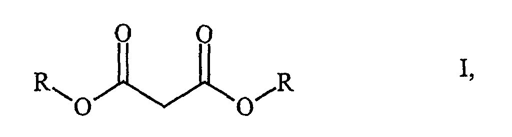 worin R C₁₋₁₀-Alkyl, C₃₋₁₀-Alkenyl oder Aryl-C₁₋₄-alkyl ist.

Die beiden gebräuchlichen Verfahren zur Herstellung von Malonsäureestern gehen von Derivaten der Chloressigsäure aus. Entweder wird ein Ester der Chloressigsäure in Gegenwart eines Katalysators auf der Basis von Cobaltcarbonyl mit Kohlenmonoxid und Alkohol umgesetzt (DE-A 23 59 963, DE-A 25 24 389) oder ein Salz der Chloressigsäure wird in einer ersten Stufe mit Cyanid zum Cyanacetat umgesetzt und dann in einer zweiten Stufe mit Alkohol in den Malonsäureester übergeführt. Insbesondere das letztgenannte Verfahren ist wegen der Toxizität von Blausäure bzw. Cyaniden und der grossen Abfallmengen sicherheitstechnisch und ökologisch bedenklich. Die an sich naheliegende Methode zur Herstellung von Estern durch direkte Veresterung der Säure mit dem entsprechenden Alkohol spielt dagegen in diesem Fall keine Rolle, vielmehr wird umgekehrt Malonsäure durch Hydrolyse von Malonsäureestern (oder Cyanessigsäure) hergestellt.

Aufgabe der vorliegenden Erfindung war die Bereitstellung eines alternativen Weges zu Malonsäurediestern.
Erfindungsgemäss wird diese Aufgabe durch das Verfahren gemäss Patentanspruch 1 gelöst.

Es wurde gefunden, dass Alkalisalze der Malonsäure in Gegenwart von Wasser mit Halogeniden der allgemeinem Formel R―X (II), worin R C₁₋₁₀-Alkyl, C₃₋₁₀-Alkenyl oder Aryl-C₁₋₄-alkyl bedeutet und X Chlor, Brom oder Iod ist, zu den entsprechenden Malonsäureestern der allgemeinen Formel 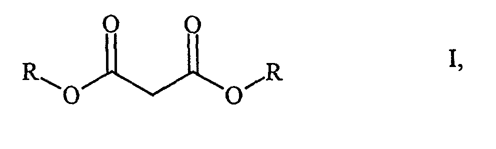 worin R die vorstehend genannte Bedeutung hat, umgesetzt werden können, wenn ein Phasentransferkatalysator anwesend ist.

Unter C₁₋₁₀-Alkyl sind hier und im folgenden alle linearen oder verzweigten primären, sekundären oder tertiären Alkyl gruppen mit 1-10 Kohlenstoffatomen zu verstehen, also beispielsweise Methyl, Ethyl, Propyl, Isopropyl, Butyl, Isobutyl, *sec*-Butyl, *tert*-Butyl, Pentyl, Isopentyl, Neopentyl, Hexyl, Heptyl, Octyl, Nonyl, Decyl usw.
Unter C₃₋₁₀-Alkenyl sind lineare oder verzweigte Alkylengruppen mit 3-10 Kohlenstoffatomen zu verstehen, insbesondere solche, deren Doppelbindung durch wenigstens ein gesättigtes Kohlenstoffatom von der freien Valenz getrennt ist, wie beispielsweise Allyl, Methallyl, 2-Butenyl (Crotyl), 3-Butenyl, 2-Pentenyl usw.
Unter Aryl-C₁₋₄-alkyl sind insbesondere phenylsubstituierte C₁₋₄-Alkylgruppen wie beispielsweise Benzyl, Phenethyl oder 3-Phenylpropyl zu verstehen, wobei die Phenylgruppe auch einen oder mehrere gleiche oder verschiedene Substituenten wie beispielsweise C₁₋₄-Alkyl, C₁₋₄-Alkoxy oder Halogen tragen kann.

Unter Phasentransferkatalysatoren sind hier und im folgenden die üblicherweise zu diesem Zweck eingesetzten Verbindungen zu verstehen, insbesondere quartäre Ammonium- oder Phosphoniumsalze.

Als Alkalisalz der Malonsäure wird vorzugsweise Dinatriummalonat eingesetzt.

Als Halogenid R―X (II) wird vorzugsweise ein Chlorid oder Bromid eingesetzt.

Das Alkalisalz der Malonsäure wird in Form einer durch katalytische Oxidation von 1,3-Propandiol in Gegenwart von wässrigem Alkalihydroxid erhaltenen wässrigen Lösung eingesetzt. Die Herstellung solcher Lösungen ist beispielsweise in DE-A 41 07 986 beschrieben.

Als Phasentransferkatalysator wird vorzugsweise ein quartäres Ammoniumsalz eingesetzt. Besonders bevorzugt sind die Tetra-*n*-C₄₋₁₀-alkylammonium-, Benzyltri-*n*-C₁₋₈-alkylammonium- und die Methyltri-*n*-C₄₋₁₀-alkylammoniumhalogenide, wobei Halogenid vorzugsweise für Chlorid oder Bromid steht. Beispielhaft seien hier Tetrabutyl- und Terahexylammoniumbromid sowie Benzyltributylammoniumchlorid genannt.

Das erfindungsgemässe Verfahren wird vorteilhaft bei Temperaturen von 80-150 °C durchgeführt, bei Einsatz niedrigsiedender Halogenide (II) zweckmässig unter erhöhten Druck.

Neben Wasser wird vorteilhaft ein mit Wasser nicht mischbares inertes Lösungsmittel eingesetzt. Hierzu eignen sich beispielsweise wenig reaktive aliphatische oder aromatische chlorierte Kohlenwasserstoffe wie Chlorbenzol oder Ether wie tert-Butylmethylether.

Die folgenden Beispiele verdeutlichen die Durchführung des erfindungsgemässen Verfahrens, ohne dass darin eine Einschränkung zu sehen ist.

### Beispiel 1

### Malonsäuredimethylester

In einem Autoklaven wurden unter Eiskühlung zu einer Lösung von 2,96 g (20 mmol) Dinatriummalonat und 0,64 g (2 mmol) Tetrabutylammoniumbromid in 5 ml Wasser 10 g (ca. 0,2 mol) Methylchlorid eingeleitet. Das Gemisch wurde innerhalb von 45 min auf 100 °C erhitzt, wobei der Druck im Autoklaven von 4 bar auf 14 bar anstieg. Nach 3 h Reaktionsdauer bei 100 °C wurde das Gemisch auf Raumtemperatur abgekühlt und entspannt. Die wässrige Phase wurde mit 1 M Natronlauge von pH 4,6 auf pH 5,7 gestellt und mit *tert*-Butylmethylether (2×10 ml) extrahiert. Die vereinigten organischen Phasen wurden über Natriumsulfat getrocknet und gaschromatographisch analysiert (interner Standard: Dimethylsuccinat).
Ausbeute: 48%.
In *tert*-Butylmethylether/Wasser (v:v = 8:5) als Reaktionsmedium wurden unter sonst gleichen Bedingungen 46% Ausbeute erhalten.

### Beispiel 2

### Malonsäurediethylester

In einem Autoklaven wurden zu einer Lösung von 2,96 g (20 mmol) Dinatriummalonat und 0,64 g (2 mmol) Tetrabutylammoniumbromid in 5 ml Wasser und 10 ml *tert-*Butylmethylether 10,9 g (0,1 mol) Ethylbromid gegeben. Das Gemisch wurde innerhalb von 30 min auf 100 °C erhitzt, wobei der Druck im Autoklaven auf 3,5 bar anstieg. Nach 3½ h Reaktionsdauer bei 100 °C wurde das Gemisch auf Raumtemperatur abgekühlt und entspannt. Die wässrige Phase wurde mit 1 M Natronlauge von pH 4,2 auf pH 5,5 gestellt und mit *tert*-Butylmethylether (2×5 ml) extrahiert. Die vereinigten organischen Phasen wurden über Natriumsulfat getrocknet und gaschromatographisch analysiert (interner Standard: Dimethylsuccinat).
Ausbeute: 45%.

### Beispiel 3

### Malonsäuredibenzylester

In einem Autoklaven wurden zu einer Lösung von 2,96 g (20 mmol) Dinatriummalonat und 0,64 g (2 mmol) Tetrabutylammoniumbromid in 5 ml Wasser und 10 ml *tert*-Butylmethylether 17,1 g (0,1 mol) Benzylbromid gegeben. Das Gemisch wurde innerhalb von 30 min auf 100 °C erhitzt, wobei der Druck im Autoklaven auf 2,5 bar anstieg. Nach 3½ h Reaktionsdauer bei 100 °C wurde das Gemisch auf Raumtemperatur abgekühlt und entspannt. Die wässrige Phase wurde mit 1 M Natronlauge von pH 1,8 auf pH 5,8 gestellt und mit tert-Butylmethylether (2×5 ml) extrahiert. Die vereinigten organischen Phasen wurden über Natriumsulfat getrocknet, das Lösungsmittel am Rotationsverdampfer abdestilliert und der Rückstand im Vakuum (1 mbar) von Lösungsmittelresten befreit.
Ausbeute: 2,43 g (43%).
¹H NMR (CDCl₃, 400 MHz): δ = 7,25-7,40 (m, 10H); 5,16 (s, 4H); 3,47 (s, 2H).
Mit Benzylchlorid statt Benzylbromid wurde unter den gleichen Reaktionsbedingungen eine Ausbeute von 34% erhalten.

### Beispiel 4

### Malonsäurediallylester

In einem Autoklaven wurden zu einer Lösung von 2,96 g (20 mmol) Dinatriummalonat und 0,64 g (2 mmol) Tetrabutylammoniumbromid in 5 ml Wasser und 10 ml Chlorbenzol 7,65 g (0,1 mol) Allylchlorid gegeben. Das Gemisch wurde innerhalb von 30 min auf 100 °C erhitzt, wobei der Druck im Autoklaven auf 2,5 bar anstieg. Nach 3½ h Reaktionsdauer bei 100 °C wurde das Gemisch auf Raumtemperatur abgekühlt und entspannt. Die wässrige Phase wurde mit *tert*-Butylmethylether (2×5 ml) extrahiert. Die vereinigten organischen Phasen wurden über Natriumsulfat getrocknet und gaschromatographisch analysiert (interner Standard: Dimethylsuccinat).
Ausbeute: 16%.

### Beispiele 5-12

### Malonsäuredimethylester

Allgemeine Vorschrift:
In einem Autoklaven wurden unter Eiskühlung zu einer Lösung von 2,96 g (20 mmol) Dinatriummalonat und 0,1 Äquivalenten (2 mmol) des Phasentransferkatalysators in 5 ml Wasser und 10 ml Chlorbenzol 10 g (0,2 mol) Methylchlorid eingeleitet. Das Gemisch wurde innerhalb von 30 min auf die gewünschte Temperatur erhitzt. Nach 3 h Reaktionsdauer bei der entsprechenden Temperatur wurde das Gemisch auf Raumtemperatur abgekühlt und entspannt. Die wässrige Phase wurde mit 1 M Natronlauge auf pH 5,5-6.5 gestellt und mit *tert*-Butylmethylether (2×10 ml) extrahiert. Die vereinigten organischen Phasen wurden über Natriumsulfat getrocknet und gaschromatographisch analysiert (interner Standard: Dimethylsuccinat). Die Reaktionsbedingungen und die erzielten Ausbeuten sind in der folgenden Tabelle 1 zusammengefasst.

**Tabelle 1**

| **Beispiel Nr.** | **Katalysator**^{**1)**} | **Temperatur [°C]** | **Reaktionszeit [h]** | **Ausbeute**^{**2)**} **[%]** |
|---|---|---|---|---|
| 5 | TBAB | 100 | 4 | 55 |
| 6 | TBAB | 100 | 6 | 56 |
| 7 | TBAB | 125 | 5 | 64 |
| 8 | TBAB | 150 | 4 | 6 |
| 9 | THAB | 100 | 4 | 58 |
| 10 | BTBACl | 100 | 4 | 39 |
| 11 | THAB | 125 | 4 | 67 |
| 12³⁾ | THAB | 100 | 4 | 21 |

| | | | | |
|---|---|---|---|---|
| ¹⁾TBAB = Tetrabutylammoniumbromid, THAB = Tetrahexylammoniumbromid, BTBACl = Benzyltributylammoniumchlorid | | | | |
| ²⁾GC, interner Standard: Dimethylsuccinat | | | | |
| ³⁾Mit Zusatz von 10 mol-% KBr | | | | |

## Patentansprüche

1. Verfahren zur Herstellung von Malonsäurediestern der allgemeinen Formel 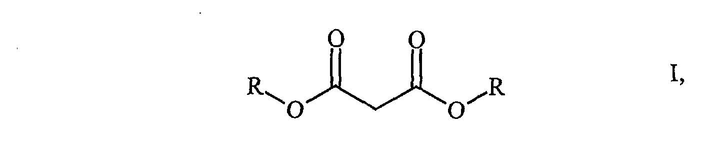 worin R C₁₋₁₀-Alkyl, C₃₋₁₀-Alkenyl oder Aryl-C₁₋₄-alkyl bedeutet, **dadurch gekennzeichnet, dass** eine durch katalytische Oxidation von 1,3-Propandiol in Gegenwart von wässrigem Alkalihydroxid erhaltene wässrige Lösung eines Alkalisalzes der Malonsäure in Gegenwart eines Phasentransferkatalysators mit einem Halogenid der allgemeinen Formel R―X (II), worin R die oben genannte Bedeutung hat und X Chlor, Brom oder Iod ist, umgesetzt wird.

2. Verfahren nach Anspruch 1, **dadurch gekennzeichnet, dass** als Alkalisalz der Malonsäure Dinatriummalonat eingesetzt wird.

3. Verfahren nach Anspruch 1 oder 2, **dadurch gekennzeichnet, dass** X Chlor oder Brom ist.

4. Verfahren nach einem der Ansprüche 1 bis 3, **dadurch gekennzeichnet, dass** als Phasentransferkatalysator ein quartäres Ammoniumsalz eingesetzt wird.

5. Verfahren nach Anspruch 4, **dadurch gekennzeichnet, dass** als quartäres Ammoniumsalz ein Tetra-*n*-C₄₋₁₀-alkylammonium-, Benzyltri-*n*-C₁₋₈-alkylammonium- oder Methyltri-*n*-C₄₋₁₀-alkylammoniumhalogenid, vorzugsweise Chlorid oder Bromid, eingesetzt wird.

## Claims

1. Process for preparing malonic diesters of the general formula 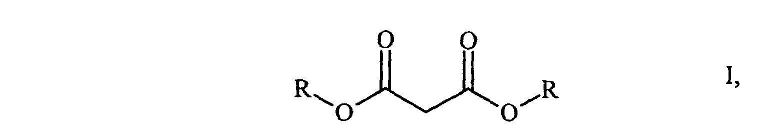 where R is C₁₋₁₀-alkyl, C₃₋₁₀-alkenyl or aryl-C₁₋₄-alkyl, **characterized in that** an aqueous solution of an alkali metal salt of malonic acid obtained by catalytic oxidation of 1,3-propanediol in the presence of aqueous alkali hydroxide is reacted with a halide of the general formula R-X (II), where R is as defined above and X is chlorine, bromine or iodine, in the presence of a phase-transfer catalyst.

2. Process according to Claim 1, **characterized in that** the alkali metal salt of malonic acid which is used is disodium malonate.

3. Process according to Claim 1 or 2, **characterized in that** X is chlorine or bromine.

4. Process according to any of Claims 1 to 3, **characterized in that** the phase-transfer catalyst used is a quaternary ammonium salt.

5. Process according to Claim 4, **characterized in that** the quaternary ammonium salt used is a tetra-*n*-C₄₋₁₀-alkylammonium, benzyltri-*n*-C₁₋₈-alkylammonium or methyltri-*n*-C₄₋₁₀-alkylammonium halide, preferably chloride or bromide.

## Revendications

1. Procédé pour la préparation de diesters d'acide malonique de formule générale 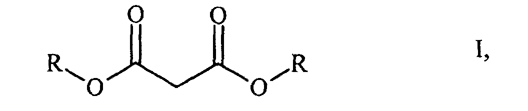 dans laquelle R représente un groupe alkyle en C₁₋₁₀, alcényle en C₃₋₁₀ ou arylalkyle(C₁₋₄), **caractérisé en ce qu'**on fait réagir une solution aqueuse d'un sel alcalin de l'acide malonique, obtenue par oxydation catalytique du 1,3-propanediol en présence d'un hydroxyde de métal alcalin aqueux, avec un halogénure de formule générale R-X (II), dans laquelle R a la signification donnée ci-dessus et X est un atome de chlore, brome ou iode, en présence d'un catalyseur de transfert de phase.

2. Procédé selon la revendication 1, **caractérisé en ce qu'**on utilise en tant que sel alcalin de l'oxyde malonique le malonate disodique.

3. Procédé selon la revendication 1 ou 2, **caractérisé en ce que** X est le chlore ou le brome.

4. Procédé selon l'une quelconque des revendications 1 à 3, **caractérisé en ce qu'**on utilise en tant que catalyseur de transfert de phase un sel d'ammonium quaternaire.

5. Procédé selon la revendication 4, **caractérisé en ce qu'**on utilise en tant que sel d'ammonium quaternaire un halogénure, de préférence un chlorure ou bromure, de tétra-*n*-alkyl(C₄₋₁₀)ammonium, benzyltri-*n*-alkyl(C₁₋₈)ammonium ou méthyltri-*n*-alkyl(C₄₋₁₀)ammonium.
